# EUROPEAN PATENT APPLICATION

(11) **EP 2 492 002 A1**
(43) Date of publication of application: **29.08.2012**
(21) Application number: 10825064.8
(22) Date of filing: 22.10.2010
(51) Int. Cl.: B01F 3/04, A23L 1/24, A23L 2/38, A47J 43/04, B01F 3/08, B01F 5/08, B01F 5/10, B01F 5/12, F04D 29/42

(54) **TREATMENT DEVICE FOR DISPERSING, DISSOLVING, COMPATIBILIZING, OR EMUSIFYING GAS/LIQUID OR LIQUID/LIQUID**

(30) Priority: 22.10.2009 JP 2009243857
(71) Applicant: EUREKA-LAB Inc., Shizuoka 425-0051 (JP)
(72) Inventor: KATAYAMA Seiji, Yaizu-shi Shizuoka 425-0051 (JP); KATAYAMA Yumiko, Yaizu-shi Shizuoka 425-0051 (JP)
(74) Representative: Piésold, Alexander James
(86) International application number: PCT/JP2010/068762
(87) International publication number: WO 2011/049215

(57) **Abstract**

It is an object of the present invention to provide a processing apparatus which is capable of performing uniform dispersion, dissolution, solubilization, emulsification of gas into liquid, and uniform dispersion, dissolution, solubilization, and emulsification of liquid to heterogeneous liquid by a large capacity and in a short time.

The invention includes a pressurizing centrifugal pump 1, a circulating portion 25 configured to circulate gas/liquid or liquid/liquid fluid from a feed port 6 to an intake port 3 of the pressurizing centrifugal pump 1, a nozzle portion 30 provided on the downstream of the pressurizing centrifugal pump 1 in the circulating portion 25 and having a minute opening configured to allow passage of the fluid pressurized by the pressurizing centrifugal pump 1, and a chamber 50 provided on the downstream of the nozzle portion 30 in the circulating portion 25 and provided with the chamber 50 which can store the fluid.

## Description

### Technical Field

The present invention relates to a processing apparatus for dispersion, dissolution and solubilization of gas into liquid, dispersion, dissolution, solubilization, and emulsification (emulsification) of heterogeneous liquid to liquid for manufacturing foods, beverages, cosmetics, drugs and medicines, sanitary materials or the like, or intended end usage in an environmental field or the like.

### Background Art

In the related art, a technique to dissolve gas such as carbonated gas, oxygen gas, hydrogen gas, or the like in liquid in drinking water such as carbonated water, oxygen water, hydrogen water is mainly performed by (1) a method of involving and mixing gas using the rotation of blades by a motor, (2) a method of spraying liquid into gas under a high-pressure, (3) a method of bubbling of gas and the like.

However, with the method of (1), it is difficult to stir and mix the gas uniformly, and hence is limited to be used in a closed system such as a static mixer. With the method of (2), a large scale facility is required for controlling the high pressure as is seen in manufacture of the carbonated water, and it is undeniable that there is demerits in terms of equipment investment or cost. The method (3) is a method of using a bubble technique as is seen in manufacture of hydrogen water on the basis of bubbling of hydrogen by Mg or electrolysis of water. However, uniform contact of air bubbles small in diameter with the liquid cannot be achieved, and hence it is difficult to obtain a uniform and stable dissolved state at high concentration.

In contrast, in manufacture of various foods, a process of uniform mixture of liquid (including gel state or viscous liquid) and heterogeneous liquid cannot be avoided. In this liquid/liquid mixture, stirring, mixing, kneading or the like using blades are mainly used for solubilization or emulsification.

However, this method depends on the method or the capability of stirring, and hence a solubilized state in which phase-separation does not occur or a fine-textured emulsified state can hardly be obtained, which significantly affects the extent (grade) of its physicality.

As a measure to cope with this problem, an advanced technique such as a high-pressure emulsification is developed in recent years and an advanced emulsification is achieved. For example, manufacture of fresh cream is achieved by stirring and mixing gas and liquid, and is an example in which its creamy emulsified state is finely affected by the method of stirring.

The inventors have worked through basic and applied studies relating to active hydrogen water having an antioxidant action and radical oxygen eliminating capability, and have tossed around the technique for dissolving the gas into the liquid in the process of studying the active hydrogen water. However, the methods in the related art as described above have both merits and demerits in terms of production efficiency, production cost, facility scale, and the like.

Accordingly, the inventors have studied about a method of dissolving gas into liquid at higher efficiency and lower cost than the method in the related art. Then, in this process, the inventors have focused on a pressurizing centrifugal pump (see Patent Documents 1 to 5).

The pressurizing centrifugal pump is configured to suck gas positively to perform pressurization and stirring simultaneously with a single pump. A cascade type pump or the like in the related art has been manufactured for the purpose of a high-discharge power, which is a pumping performance, and hence cavitation caused by entraining of gas is hated. In contrast, the pressurizing centrifugal pump positively enables mixing of gas by the force of impulsive waves of the cavitation, and is developed as a groundbreaking pump having an enhanced discharge power as much as approximately three times that in the related art.

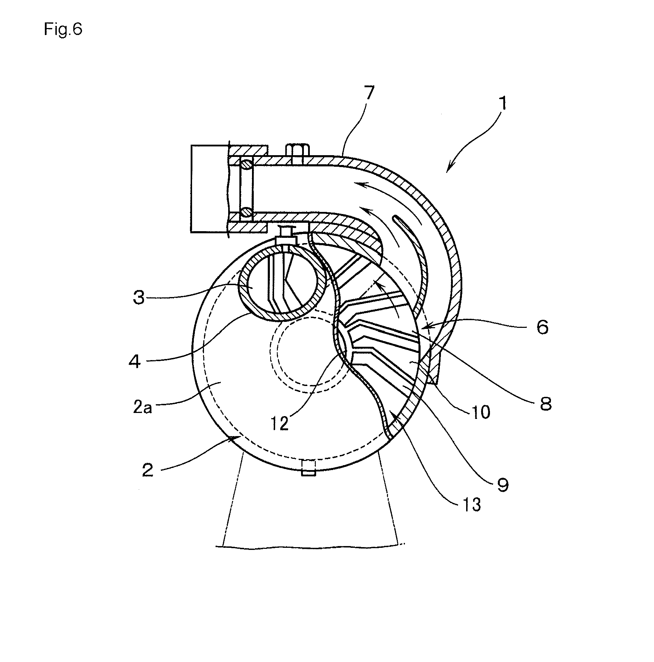

Fig. 6 to Fig. 8 illustrate an example of the pressurizing centrifugal pump. A pressurizing centrifugal pump 1 includes a case 2 having a drum shape and including an intake port 3 and a feed port 6 as shown in Fig. 6 to Fig. 7, and the case 2 includes a pressurizing case 2a having the intake port 3 and an impeller case 2b having the feed port 3 on the left side and the right side as a pair.

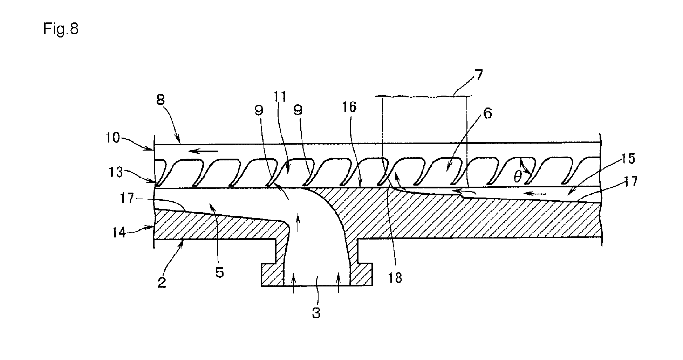

The pressurizing case 2a includes a pressurizing portion 14 formed integrally with a case lid portion having an intake pipe 4, the pressurizing portion 14 fitting into an opening of the impeller case 2b in a state an impeller 8 is assembled thereto, and the pressurizing case 2a and the impeller case 2b are fixedly fastened by a fixture, whereby the case 2 is formed in a closed state. Accordingly, a pump chamber 13 (a pressurizing chamber 15 in Fig. 8) for pressurizing fluid sucked from the intake port 3 via the impeller 8 and feeding out from the feed port 6 is formed between the pressurizing portion 14 and the impeller 8.

The impeller case 2b is integrally formed with a peripheral wall having a width which allows fitting of the impeller 8 and the pressurizing portion 14 of the pressurizing case 2a on the outer periphery of a disk-shaped side wall. On the peripheral wall, the feed port 6 having a predetermined length extending across plural blades 9, 9, ... is formed at a predetermined portion opposing the blade width of the impeller 8. Then, a feed pipe 7 curved in the direction of feeding the fluid is integrally connected to the feed port 6.

The side wall of the impeller case 2b is integrally coupled with a supporting portion on the outside thereof, and rotatably supports a pump shaft by placing the same at a center portion of the pump chamber 13.

The impeller 8 is integrally formed with a cylindrical boss portion 12 which also serves as a mounting member with respect to the pump shaft from a center portion of a disk-shaped blade plate 10, which corresponds to the blade-side wall.

Then, the respective blades 9 are projected radially from the blade plate 10 and the boss portion 12 at predetermined intervals, and a space portion formed by the respective blades 9, the blade plate 10 and the boss portion 12 correspond to impeller chambers 11 which contain the fluid in the interior thereof.

The boss portion 12 and side ends of the blades 9 of the impeller 8 are formed at substantially the same level, so that end surface of the boss portion 12 is brought into proximity to end surface of a flat shaped partitioning wall 19 formed at a center portion of the pressurizing case 2a when being mounted on the impeller case 2b, and an abrasion resistant member is interposed between the both members for shielding.

The blades 9 of the impeller 8 are projected radially from the boss portion 12 toward the upstream side in the direction of rotation of the impeller on one side surface of the disk-shaped blade plate 10, and the flat-panel shaped blade strips are bent at midsections of the length thereof so as to be tilted rearward in side view.

In addition, in order to cause an outer end surface (the thicker end) of the blades 9 on the side of the pressurizing case 2a to move prior to the proximal side of the blade plate 10, a forward tilting angle (rake angle) θ is provided as shown in Fig. 8 so as to be inclined toward the downstream side in the direction of rotation of the impeller 8.

With this blade shape, the fluid can easily be raked up from the intake port 3 along with the rotation of the impeller 8, whereby the fluid is held in the impeller chamber 11. Then, the respective blades 9 push out and urge the fluid in the impeller chambers 11 as if causing the same to kick while applying a centrifugal force by the blade shape tilted rearward when reaching the portion of the feed port 6, thereby enhancing the flow pressure in the centrifugal direction and increasing the feeding efficiency of the fluid.

Then, as shown in Fig. 8, the pump chamber 13 includes an intake chamber 5 which promotes sucking of the fluid and the pressurizing chamber 15 communicating therewith and pressurizing the fluid.

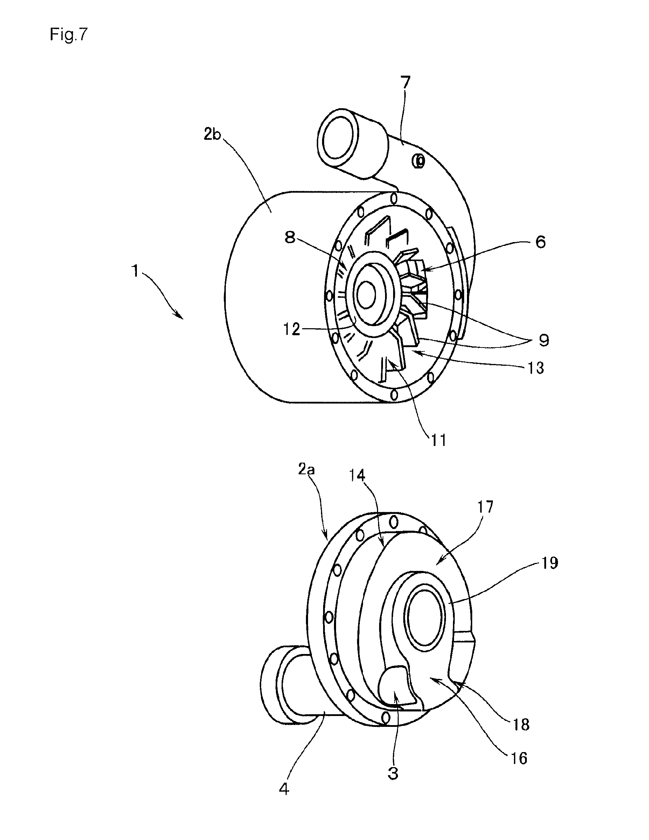

Also, formed between a terminal end of the pressurizing chamber 15 and the intake port 3 is a pressurizing partitioning wall 16 which is in the proximity to side surfaces of the plural blades 9 and restricts the leakage of the fluid in the impeller chambers 11 so as to have a flat shape flush with the partitioning wall 19 in Fig. 7. Accordingly, formed around the partitioning wall 19 opposing the end surface of the boss portion 12 of the impeller 8 is a series of the intake chamber 5, the pressurizing chamber 15, and the pressurizing partitioning wall 16.

Also, a pressurizing plane 17 formed of a smooth tilted surface in a range from the intake port 3 to the pressurizing partitioning wall 16 forms the pressurizing chamber 15 gradually approaching the blades 9 from the side of the intake chamber 5 in a converging manner. Accordingly, the fluid sucked from the intake port 3 into the pump chamber 13 is pressurized gradually by the plural blades 9 via the pressurizing chamber 15 as a long channel in a state of being raked and held in the respective impeller chambers 11 in sequence by the rotation of the impeller 8.

The pressurizing plane 17 is formed to a pressurization terminal point 18 which is located at a starting end of the pressurizing partitioning wall 16, and pressurizes and guides the fluid moving from the intake chamber 5 toward the downstream side into the impeller chambers 11 by causing the same to flow along the pressurizing plane 17. Also, the fluid is pressurized in the chamber 13 without causing abrupt pressure fluctuations and the fluid pressurized to a maximum pressure at the position of the pressurization terminal point 18 is efficiently pushed out from the feed port 6.

When one side of the pump shaft is driven from the side of a power engine and rotates the impeller 8 in the direction indicated by an arrow, the respective blades 9 rape up and suck the fluid and the air from the intake port 3 into the impeller chambers 11 and hold and turn the fluid in a state of being stored in the respective impeller chambers 11 and bring the same continuously into the pump chamber 13. Then, the fluid and the air bubbles in the pressurizing chamber 15 are pressurized along the pressurizing plane 17 and enter the impeller chamber 11 while increasing in pressure thereof and reach the pressurizing partitioning wall 16, then become a most pressurized state and are added with a pushing-out force and a centrifugal force caused by the shape of the pressurizing plane 17 and the rotation of the blades 9, and fed out from the feed port 6.
[Patent Document 1] JP-A-2001-159398
[Patent Document 2] JP-A-2002-089477
[Patent Document 3] JP-A-2004-060470
[Patent Document 4] JP-A-2005-290999
[Patent Document 5] JP-A-2008-038619

### Disclosure of Invention

### Problems to be Solved by the Invention

The pressurizing centrifugal pump 1 is developed for the purpose of obtaining a high pumping performance, that is, a high discharging amount by positively involving gas. However, the inventors focused on the actions of pressurization, compression, stirring, mixing, centrifugation, impulse waves of cavitation in the pump, and studied the processing apparatus which enables efficient dispersion, dissolution, solubilization, and emulsification of gas into liquid by applying this technique and, in addition, enables dispersion, dissolution, solubilization, and emulsification of the liquid to heterogeneous liquid.

In view of such circumstances as described above, it is an object of the present invention to provide a processing apparatus which is capable of performing uniform dispersion, dissolution, solubilization, emulsification of gas into liquid, and uniform dispersion, dissolution, solubilization, and emulsification of liquid to heterogeneous liquid by a large capacity and in a short time.

### Means for Solving the Problem

In order to solve the above-described problem, the invention has following characteristics.

First: A processing apparatus for dispersion, dissolution, solubilization, or emulsification of gas/liquid or liquid/liquid configured to disperse, dissolve, solubilize, or emulsify gas into liquid or disperse, dissolve, solubilize, or emulsify liquid to liquid, including:
a pressurizing centrifugal pump having a pump chamber in a drum-shaped case formed with an intake port and a feed port, an impeller having plural blades projecting radially from a boss portion on a side surface of a blade plate so as to have a retracted angle in the direction of rotation, a pressurizing portion having a pressurizing plane which opposes blades and forms a pressurizing chamber converging from the side of the intake port toward the side of the feed port and a pressurizing partitioning wall provided in the proximity of side surfaces of the blades and configured to prevent leakage of the fluid in an impeller chamber, the pressurizing portion opposing the impeller,
a circulating portion communicating with an intake port and a feed port of the pressurizing centrifugal pump, configuring the circulating channel together with the pressurizing centrifugal pump, and causing gas/liquid fluid or liquid/liquid fluid to be dispersed, dissolved, solubilized, or emulsified to be circulated by driving of the pressurizing centrifugal pump from the feed port to the intake port of the pressurizing centrifugal pump,
a nozzle portion provided on the downstream of the pressurizing centrifugal pump in the circulating portion and having a minute opening for allowing gas/liquid fluid or liquid/liquid fluid pressurized by the pressurizing centrifugal pump to pass therethrough, and
a chamber provided on the downstream of the nozzle portion in the circulating portion and configured to be capable of storing gas/liquid fluid or liquid/liquid fluid.

Second: The first processing apparatus for gas/liquid or liquid/liquid dispersion, dissolution, solubilization, or emulsification described above including a valve which can adjust the width of the minute opening of the nozzle portion by a valve body.

### Advantages of the Invention

According to the present invention, the nozzle portion configured to allow the fluid pressurized by the pressurizing centrifugal pump to pass therethrough and the chamber configured to be capable of storing the fluid passed through the nozzle portion, whereby the fluid is caused to circulate through the pressurizing centrifugal pump, the nozzle portion, and the chamber.

When the pressurizing centrifugal pump is driven, in the pump, the fluid is discharged from the feed port under the pressurized state upon being subject to actions such as pressurization, compression, stirring, mixing, centrifugation, cavitation, and the like. The gas/liquid or liquid/liquid fluid dispersion, dissolution, solubilization, or emulsification of the discharged fluid is further encouraged upon being subject to pressurization, compression, change in flow velocity or the like by the action of the nozzle portion at the downstream of the pressurizing centrifugal pump, and foam is typically generated. Then, the fluid passed through the minute opening of the nozzle portion is received once in the large-capacity chamber as a pool for controlling the pressure and hence is alleviated in pressure, whereby the fluid is stably uniformized. Furthermore, the fluid in the chamber is returned to the intake port of the pressurizing centrifugal pump by the circulating channel, and the process described above is repeated. Accordingly, uniform dispersion, dissolution, solubilization, and emulsification of the gas/liquid or liquid/liquid fluid is achieved.

In this manner, the nozzle portion and the chamber cooperate with the function of the pressurizing centrifugal pump, and act to enhance the efficiency of dispersion, dissolution, solubilization, emulsification, and hence the compressed bubble release or the like is enabled. In other words, according to the present invention, the pressurization, compression, stirring, mixing, centrifugation, cavitation and bubbling or the like can be performed simultaneously and efficiently in one process, whereby uniform dispersion, dissolution and solubilization of gas into liquid, and uniform dispersion, dissolution, solubilization, and emulsification of liquid such as gel or viscous liquid or the like into heterogeneous liquid can be performed by a large capacity and in a short time.

Therefore, the processing apparatus according to the present invention enables simplification of production facilities, energy saving, high production efficiency, and reduction of production cost or the like in various fields such as foods, beverages, cosmetics, drugs and medicines, sanitary materials, environments.

### Brief Description of Drawings

[Fig. 1] Fig. 1 is a drawing showing an embodiment of a processing apparatus according to the present invention.
[Fig. 2] Fig. 2 is a cross-sectional view showing a valve used in the processing apparatus according to the present invention.
[Fig. 3] Fig. 3 is a graph showing a relation between the height and time of an emulsified state in Example 5.
[Fig. 4] Fig. 4 is a graph showing a relation between the height and time of an emulsified state in Example 6.
[Fig. 5] Fig. 5 is a graph showing changes with time of amount of dissolved hydrogen, pH, oxidation-reduction potential (ORP) of hydrogen water manufactured in Example 7 in a state of being opened to the atmospheric air.
[Fig. 6] Fig. 6 is a side view, partly broken, showing a pressurizing centrifugal pump.
[Fig. 7] Fig. 7 is an exploded perspective view showing a case structure of the pressurizing centrifugal pump.
[Fig. 8] Fig. 8 is a cross-sectional view showing a configuration of a pump chamber of the pressurizing centrifugal pump.

### Reference Numerals

- 1: pressurizing centrifugal pump
- 2: case
- 2a: pressurizing case
- 2b: impeller case
- 3: intake port
- 4: intake pipe
- 5: intake chamber
- 6: feed port
- 7: feed pipe
- 8: impeller
- 9: blade
- 10: blade plate
- 11: impeller chamber
- 12: boss portion
- 13: pump chamber
- 14: pressurizing portion
- 15: pressurizing chamber
- 16: pressurizing partitioning wall
- 17: pressurizing plane
- 18: pressurization terminal point
- 19: partitioning wall
- θ: blade forward tilting angle
- 20: processing apparatus
- 25: circulating portion
- 26: circulating channel
- 30: nozzle portion
- 31: minute opening
- 35: valve
- 36: valve body
- 37: Venturi tube
- 38: fluid inlet port
- 39: fluid outlet port
- 40: valve body
- 50: chamber
- 51: safety valve
- 60: liquid sample storage tank
- 61: gas sample storage tank
- 65: mixer
- 66: mixer
- 70: flow-channel switching valve
- 71: flow-channel switching valve
- 80: storage tank
- 81: takeout port

### Best Mode for Carrying Out the Invention

Referring now to the drawings, an embodiment of the present invention will be described.

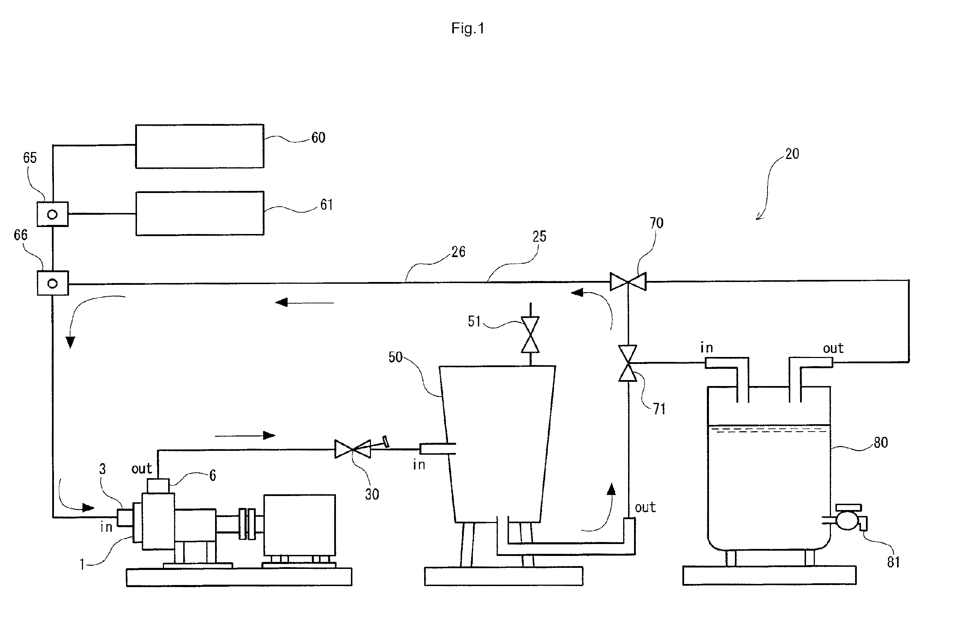

Fig. 1 is a drawing schematically showing an embodiment of a processing apparatus for dispersion, dissolution, solubilization, or emulsification of gas/liquid, or liquid/liquid (hereinafter, referred to as "processing apparatus").

As shown in Fig. 1, a processing apparatus 20 of the embodiment includes a pressurizing centrifugal pump 1 and a circulating portion 25 communicating therewith. The circulating portion 25 communicates with an intake port 3 and a feed port 6 of the pressurizing centrifugal pump 1, and constitutes a circulating channel 26 together with the pressurizing centrifugal pump 1.

Provided on the downstream of the pressurizing centrifugal pump 1 of the circulating portion 25 is a nozzle portion 30 further on the downstream of the nozzle portion 30 of the circulating portion 25 is a chamber 50.

As the pressurizing centrifugal pump 1, the one known in the related art can be used and, for example, the one having configurations described in Patent Documents 1 to 5, may be used. In this embodiment, the one having configurations shown in Fig. 6 to Fig. 8 is used.

In other words, as shown in Fig. 6 to Fig. 8, the pressurizing centrifugal pump 1 includes a pump chamber 13 in a drum-shaped case 2 having the intake port 3 and the feed port 6, in which a pressurizing portion 14 having a pressurizing plane 17 which opposes blades 9 and forms a pressurizing chamber 15 and is converged from the side of the intake port 3 toward the side of the feed port 6 and a pressurizing partitioning wall 16 provided in the proximity of a side surface of the blades 9 and configured to prevent leakage of fluid in an impeller chamber 11 are provided so as to oppose an impeller 8 having the plural blades 9 projecting radially so as to have a retracted angle in the direction of rotation from a boss portion 12 on side surfaces of blade plates 10. Then, when the pressurizing centrifugal pump 1 is activated to rotate the impeller 8 in the direction indicated by an arrow, the respective blades 9 rake up and suck the fluid and gas from the intake port 3 into the impeller chambers 11 and hold and turn the fluid in a state of being stored in the respective impeller chambers 11 and bring the same continuously into the pump chamber 13. Then, the fluid and air bubbles in the pressurizing chamber 15 are pressurized along the pressurizing plane 17 and enter the impeller chamber 11 while increasing in pressure thereof and reach the pressurizing partitioning wall 16, then become a most pressurized state and are added with a pushing-out force and a centrifugal force caused by the shape of the pressurizing plane 17 and the rotation of the blades 9, and fed out from the feed port 6.

In the circulating portion 25 in Fig. 1 which communicates with the pressurizing centrifugal pump 1, the circulating channel 26 may be formed of a conduit such as a pipe formed of a suitable material such as metals or the like considering the fluid pressure or the like.

The nozzle portion 30 provided on the downstream of the pressurizing centrifugal pump 1 has a minute opening which allows passage of the fluid pressurized by the pressurizing centrifugal pump 1, and the fluid pressurized by the pressurizing centrifugal pump 1 is subject to pressurization, compression, and change in flow velocity or the like at the nozzle portion 30 and hence foam typically, whereby dispersion, dissolution, solubilization or emulsification of the fluid are encouraged.

The nozzle portion 30 in the present invention has a general action as a nozzle, that is, an action to control the characteristics of the fluid such as flow rate, flow velocity, direction and pressure, and is not necessarily limited to be a tubular shape and is to be broadly interpreted as long as it has a minute opening to discharge the fluid (has a configuration which causes the fluid to pass through a narrow hole). For example, a gap between a valve body and a valve seat surface of the valve is included, which allow adjustment of the width of the minute opening by the valve body. Examples of the valve described above include, for example, needle valves, gate valves, glove valves, ball valves, port valves, and butterfly valves or the like.

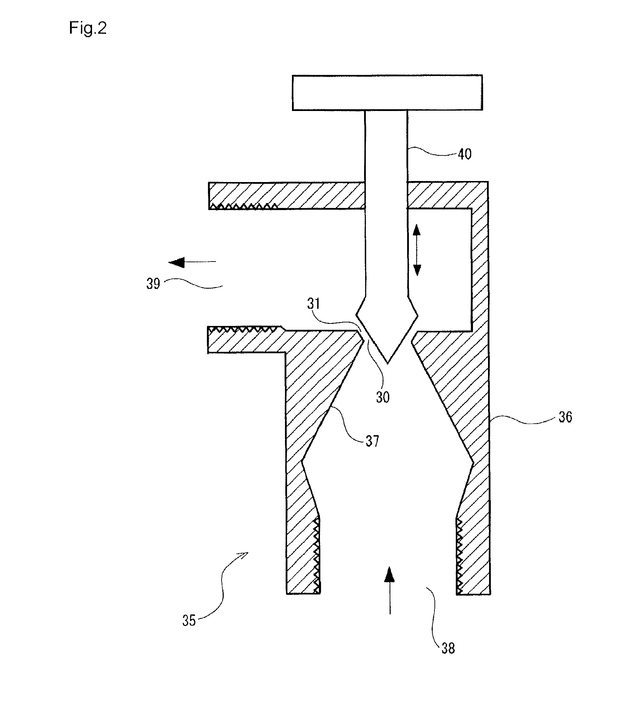

As a configuration suitable for generating micro bubbles on the basis of the compressed bubble release, a valve 35 shown in Fig. 2 may be employed, for example. The valve 35 is provided with a Venturi tube 37 which is reduced in diameter in a tapered shape along the direction of inflow of the fluid from a fluid inlet port 38 in the interior of a valve body 36 having the fluid inlet port 38 and a fluid outlet port 39 formed so as to cause fluid to flow in the direction perpendicular, a valve element 40 provided at a rear end portion of the Venturi tube 37 so as to be capable of moving back and forth in this direction, whereby the nozzle portion 30 having a minute opening 31 as a gap between the valve element 40 and the rear end portion of the Venturi tube 37 is achieved. The needle-shaped valve element 40 configuring a gate valve approaches the surface of the rear end portion of the Venturi tube 37 from the side opposite from the flow channel, so that the nozzle diameter (the width of the minute opening 31) is controlled.

The fluid discharged from the pressurizing centrifugal pump 1 in Fig. 1 flows from the fluid inlet port 38 of the valve 35 in Fig. 2, flows from a wider diameter toward a narrower diameter of the tapered Venturi tube 37, passes through the minute opening 31 having a narrower diameter at a distal end of the taper, and is discharged from the fluid outlet port 39.

When the fluid passes through the taper of the Venturi tube 37, the flow velocity (the dynamic pressure of a pressure component in the direction of the flow velocity) is decreased and the static pressure (the pressure component perpendicular to the direction of the flow velocity) is increased when the fluid passes through the larger diameter, while the flow velocity is increased and the static pressure is decreased when the fluid passes through the smaller diameter on the basis of the Bernoulli's theorem. Therefore, when the fluid passes through the larger diameter, the gas is under the conditions of being dissoluble in liquid, and when the fluid passes through the smaller diameter, the gas is in the state of being hardly dissoluble in liquid, thereby generating air bubbles.

The air bubbles generated in the vicinity of the nozzle portion 30 are encouraged to become finer bubbles by a shear stress and a pressure difference (an abrupt decrease in dynamic pressure) when sneaking through the minute opening 31 having further narrower diameter. Therefore, the diameter of the bubble is determined depending on the difference in diameter of the taper, and the diameters of the bubbles or the state of distribution can be controlled according to the extent of screwing-in of the needle-shaped valve element 40.

The chamber 50 in Fig. 1 serves as a pool for controlling the pressure, receives the fluid discharged from the minute opening of the nozzle portion 30 once, alleviates the pressure, and stably uniformizes the fluid. A lid member of the chamber 50 is provided with a safety valve 51, so that the pressure in the sealed chamber 50 can be released by opening the safety valve 51.

Also, in this embodiment, as a configuration of dispersing, dissolving, solubilizing or emulsifying gas into liquid, for example, as a configuration of dispersing, dissolving, solubilizing or emulsifying liquid such as water to gas such as O₂, N₂, CO₂, or H₂, a liquid sample storage tank 60 and a gas sample storage tank 61 are connected to the circulating portion 25 via mixers 65, 66 to introduce the liquid 60 stored in the liquid sample storage tank 60 and the gas stored in the gas sample storage tank 61 into the circulating portion 25.

The liquid sample storage tank 60 and the gas sample storage tank 61 are provided as needed and, for example, the liquid may be introduced directly into the circulating portion 25 from the supply source.

Introduction of the gas into the pressurizing centrifugal pump 1, for example, may be achieved by self supply of the pressurizing centrifugal pump 1 or through a gas mixing hole provided on the pressurizing centrifugal pump 1.

Also, when dispersing, dissolving, solubilizing or emulsifying liquid to heterogeneous liquid, for example, when dispersing, dissolving, solubilizing or emulsifying two or more types of liquid such as water, oil, solvent, and the like, a configuration in which the plural liquid sample storage tanks 60 having these types of liquid stored therein are connected is also applicable for example.

The mixers 65, 66 may have a configuration in which a flow channel having a check valve can be merged with another flow channel.

Also, in this embodiment, a storage tank 80 for sample after the process is connected to the circulating portion 25 via flow-channel switching valves 70, 71 and the sample after the process is stored therein so as to be taken out from a takeout port 81 arbitrarily.

Using the processing apparatus 20 in this embodiment described above, dissolution, solubilization, and emulsification of gas into liquid are performed as follows.

Liquid sample and gas sample are flowed into the circulating portion 25 via the mixers 65, 66 from the liquid sample storage tank 60 and the gas sample storage tank 61, respectively, and the gas/liquid fluid is circulated in the circulating portion 25 by driving the pressurizing centrifugal pump 1.

At the time of this circulation, in the pressurizing centrifugal pump 1, the fluid is discharged from the feed port 6 under the pressurized state upon being subject to actions such as pressurization, compression, stirring, mixing, centrifugation, cavitation, and the like.

The gas/liquid dispersion, dissolution, solubilization, and emulsification of the discharged fluid are further encouraged upon being subject to pressurization or change in flow velocity or the like by the action of the nozzle portion 30 of the pressurizing centrifugal pump 1 on the downstream thereof, and fine foam is typically generated. For example, after the activation of the pressurizing centrifugal pump 1, the valve element 40 of the valve 35 as shown in Fig. 2 is moved to reduce the minute opening 31 of the nozzle portion 30, the fluid pressure between the pressurizing centrifugal pump 1 and the nozzle portion 30 is abruptly increased, and reaches equilibrium in a pressurized state. Then, the compressed bubble release occurs at the nozzle portion 30.

The fluid after foaming passed through the minute opening of the nozzle portion 30 is received once in the large-capacity chamber 50 as a pool for controlling the pressure and hence is alleviated in pressure, whereby the gas/liquid dispersion, dissolution, solubilization, and emulsification are encouraged and hence the fluid is stably uniformized.

Furthermore, the fluid in the chamber 50 is returned to the intake port 3 of the pressurizing centrifugal pump 1 by the circulating channel 26, and the process described above is repeated.

In this manner, the nozzle portion 30 and the chamber 50 cooperate with the function of the pressurizing centrifugal pump 1, and act to enhance the efficiency of dispersion, dissolution, solubilization and emulsification. In other words, the pressurization, compression, stirring, mixing, centrifugation, cavitation, and bubbling or the like can be performed simultaneously in one process and efficiently, whereby the uniform dispersion, dissolution, solubilization, and emulsification of gas into liquid can be performed by a large capacity and in a short time.

Then, by replacing the sample with a liquid/liquid system, the uniform dispersion, dissolution, solubilization, and emulsification of liquid such as gel or viscous liquid to heterogeneous liquid can be performed by a large capacity and in a short time by the similar action as that in the case of the gas/liquid system described above.

The processing apparatus according to the present invention enables uniform dispersion, dissolution, solubilization, and emulsification of gas/liquid, liquid/liquid and, for example, may be used for manufacturing foods such as fresh cream, butter, mayonnaise, dressing, juice, ice cream, homogenized milk, jelly, and the like.

Also, the invention enables uniform mixture of gas/liquid, liquid/liquid and may be used, for example, for manufacturing aerosol such as aromatic substance, or cosmetics such as emulsion, cream, gel.

Also, the invention facilitates manufacture of carbonated water, oxygen water, hydrogen water, ozone water, and the like and, in particular, may be used for manufacturing healthy drinks such as mineral water, sport drink, jelly drink.

Also, the invention may be used for manufacturing infusion solution having an antioxidant potential and a radical oxygen eliminating potential such as reduced hydrogen water or drugs and medicines such as drug solution.

Also, the invention may be used for manufacturing sanitary materials intended for antibacterial properties, sterilization properties, and mold-free properties such as manufacture of ozone water controlled in concentration, or an application in an environmental field.

Also, the invention may be used in an application to an environmental field which contributes to energy saving and reduction of CO₂ release by manufacturing emulsion fuel formed of light gas oil/air or light gas oil/air/water.

Also, the invention may be used in an application to an environmental field which contributes to energy saving and reduction of CO₂ release by manufacturing emulsion fuel formed of heavy oil (C)/water or heavy oil/water/air.

### Examples

Although the present invention will be described further in detail with reference to examples below, the present invention is not limited to these examples.

### <Example 1 >

### Manufacture of Carbonated Water

A pressurizing centrifugal pump (manufactured by Yonehara Gikensha) having a motor of 3.7 kw mounted thereon, a valve provided with a nozzle portion with a Venturi tube as shown in Fig. 2 and a chamber were connected using a stainless pipe (SUS32) as shown in Fig. 1 to configure a circulating portion in which sample liquid was to be circulated.

By using this processing apparatus, the chamber was filled with 30L of water, and the apparatus was activated at three-phase 200V, a frequency of 60Hz via an inverter at the number of revolutions of 4000.

The circulating flow rate was set to approximately 100 L/min, the diameter of the nozzle portion of the valve was adjusted by a valve body, and the static pressure on the discharging side of the pressurizing centrifugal pump was maintained at 0.4 MPa.

The circulating portion was filled with carbonated gas (CO₂) at a flow rate of 3L/min from a gas sample storage tank on the side of an air-inlet port of the pressurizing centrifugal pump. Accordingly, the carbonated gas and the water were subjected to compression, stirring and the like in the pressurizing centrifugal pump and were discharged toward the chamber, foamed via the nozzle portion with the Venturi tube at a midsection, and reached the chamber and, in this process, the carbonated gas was dissolved in the water.

In this example, the circulation was performed under the conditions of being opened to the atmospheric air in a state in which a lid of the chamber was opened. Under such conditions, part of the dissolved carbonated gas can escape to the atmospheric air. The sample passed through the chamber and returned back to the pressurizing centrifugal pump, while carbonated gas was replenished again from the gas sample storage tank immediately before the pump. This cycle is determined as a circulation, so that 30L of sample liquid circulates approximately 3.3 times per minute.

The amount of the carbonated gas dissolved in water was estimated from a standard curve by measuring pH of the solution (HORIBA pH METER F-52). The result is shown in Table 1.

**[Table 1]**

| Min | pH | *µ* g/L |
|---|---|---|
| 0 | 7.9 | 0.5 |
| 2.5 | 7.0 | 3.0 |
| 5.0 | 5.0 | 35 |
| 10.0 | 4.7 | 60 |
| 12.5 | 4.4 | 150 |
| 15.0 | 4.1 | 300 |
| 20.0 | 4.1 | 300 |

In this manner, it became apparent that the dissolved amount of the carbonated gas was gradually increased along with the circulation of the sample liquid, and after approximately 15 minutes, that is, in a state in which 30L of sample circulated by approximately 50 times, the equilibrium condition was achieved. By driving the camber in the sealed state, escape of the carbonated gas to the atmospheric pressure eliminated, and manufacture of carbonated water at a high concentration with higher efficiency is enabled.

### [Example 2]

### Manufacture of Oxygen Water

By using the same processing apparatus as in Example 1, the chamber was filled with 30L of water, and the apparatus was activated at three-phase 200V, a frequency of 60Hz via the inverter at the number of revolutions of 4000.

The circulating flow rate was set to approximately 100 L/min, the diameter of the nozzle portion of the valve was adjusted by the valve body, and the static pressure on the discharging side of the pressurizing centrifugal pump was maintained at 0.4 MPa.

The circulating portion was filled with oxygen gas (O₂) at a flow rate of 2L/min from the gas sample storage tank on the side of an air-inlet port of the pressurizing centrifugal pump. Accordingly, the oxygen gas and the water were subjected to compression, stirring and the like in the pressurizing centrifugal pump and were discharged toward the chamber, foamed via the nozzle portion with the Venturi tube at the midsection, and reached the chamber and, in this process, the oxygen gas was dissolved in the water.

The amount of the oxygen gas dissolved in water was estimated from a standard curve by measuring pH of the solution (HORIBA DO MEER OM-51). The result is shown in Table 2.

**[Table 2]**

| Min | *µ*g/L |
|---|---|
| 0 | 6.1 |
| 2.0 | 8.6 |
| 5.0 | 11.1 |
| 7.5 | 12.5 |
| 10.0 | 12.7 |
| 12.5 | 13.7 |
| 15.0 | 16.6 |
| 18.0 | 19.9 |
| 30.0 | 19.9 (scale over) |

In this manner, the dissolved amount of the oxygen gas was gradually increased along with the circulation of the sample liquid, and after approximately 18 minutes, the scale over of a gauge was resulted. The dissolution equilibrium condition seemed to be still to come. In this example, the circulation was performed under the conditions of being opened to the atmospheric air in a state in which the lid of the chamber was opened. However, it is estimated that manufacture of oxygen water at a higher concentration is enabled by driving the chamber in a sealed state and increasing the amount of infused oxygen.

### <Example 3>

### Manufacture of Hydrogen Water

By using the same processing apparatus as in Example 1, the chamber was filled with 30L of water, and the apparatus was activated at three-phase 200V, a frequency of 60Hz via the inverter at the number of revolutions of 4000.

The circulating flow rate was set to approximately 100 L/min, the diameter of the nozzle portion of the valve was adjusted by the valve body, and the static pressure on the discharging side of the pressurizing centrifugal pump was maintained at 0.3 MPa.

The circulating portion was filled with hydrogen gas (H₂) at a flow rate of 1.5L/min from the gas sample storage tank on the side of an air-inlet port of the pressurizing centrifugal pump. Accordingly, the hydrogen gas and the water were subjected to compression, stirring and the like in the pressurizing centrifugal pump and were discharged toward the chamber, foamed via the nozzle portion with the Venturi tube at the midsection, and reached the chamber and, in this process, the hydrogen gas was dissolved in the water. The water temperature at the time of start of the experiment was 25°C, and the water temperature at the time of end after 10 minutes was 32°C.

The amount of the hydrogen gas dissolved in the water was measured by using KM2100 DH manufactured by KYOEI DENSHI KENKYUJO, and the value of the oxidation-reduction potential (ORP) at that time was measured using HORIBA ORP METER F-52. The result is shown in Table 3.

**[Table 3]**

| Min | H₂ (*µ* g/L) | ORP(mV) |
|---|---|---|
| 0 | 0.0 | 0.0 |
| 2.0 | 0.05 | -58.0 |
| 4.0 | 0.28 | -98.0 |
| 6.0 | 0.43 | -171.0 |
| 8.0 | 0.72 | -182.0 |
| 10.0 | 0.73 | -187.0 |

In this manner, it became apparent that the dissolved amount of the hydrogen gas was gradually increased along with the circulation of the sample liquid, and after approximately 10 minutes, the dissolution equilibrium was achieved. In this example, the circulation was performed under the conditions of being opened to the atmospheric air in a state in which the lid of the chamber was opened. However, it is estimated that manufacture of hydrogen water at a higher concentration with high degree of efficient is enabled by driving the chamber in a sealed state so as to reduce the escape of the hydrogen gas to the atmospheric air.

### <Example 4>

### Manufacture of Light Gas Oil/Water/Air Emulsion

By using the same processing apparatus as in Example 1, the chamber was filled with 30L of light gas oil, approximately 100 mL of water, and the apparatus was activated at three-phase 200V, a frequency of 60Hz via an inverter at the number of revolutions of 4000.

The circulating flow rate was set to approximately 100 L/min, the diameter of the nozzle portion of the valve was adjusted by the valve body, and the static pressure on the discharging side of the pressurizing centrifugal pump was maintained at 0.4 MPa.

The circulating portion was filled with air at a flow rate of 3L/min from the gas sample storage tank on the side of an air-inlet port of the pressurizing centrifugal pump. Accordingly, the light gas oil and the water and the air were subjected to compression, stirring and the like in the pressurizing centrifugal pump and were discharged toward the chamber, foamed via the nozzle portion with the Venturi tube at the midsection, and reached the chamber and, after 2 to 3 minutes of repetition of this circulation, the light gas oil and the water and the air were turned into emulsion and opacified.

This opacified state was stably kept for a period on the order of half a day. In contrast, the emulsion manufactured by stirring (hard shaking manually for about 10 minutes) without employing the emulsification on the basis of the processing apparatus of this example returned back to its original phase dissociated state after the elapse of time for about 10 minutes. Therefore, it is estimated that finer vesiculation (emulsification) of light gas oil and water and air has occurred by a mechanical force of the impeller by the high-speed rotation of the pressurizing centrifugal pump, that is, by the shear stress thereof. It is estimated that the constituent of emulsion is formed by cloudy emulsification of micelle of w/o and air bubbles.

### <Example 5>

### Manufacture of Dressing

By using the same processing apparatus as in Example 1, the chamber was filled with 20L of edible formula oil and 10L of edible vinegar, and the apparatus was activated at three-phase 200V, a frequency of 60Hz via the inverter at the number of revolutions of 4000.

The circulating flow rate was set to approximately 100 L/min, the diameter of the nozzle portion of the valve was adjusted by the valve body, and the static pressure on the discharging side of the pressurizing centrifugal pump was maintained at 0.4 MPa.

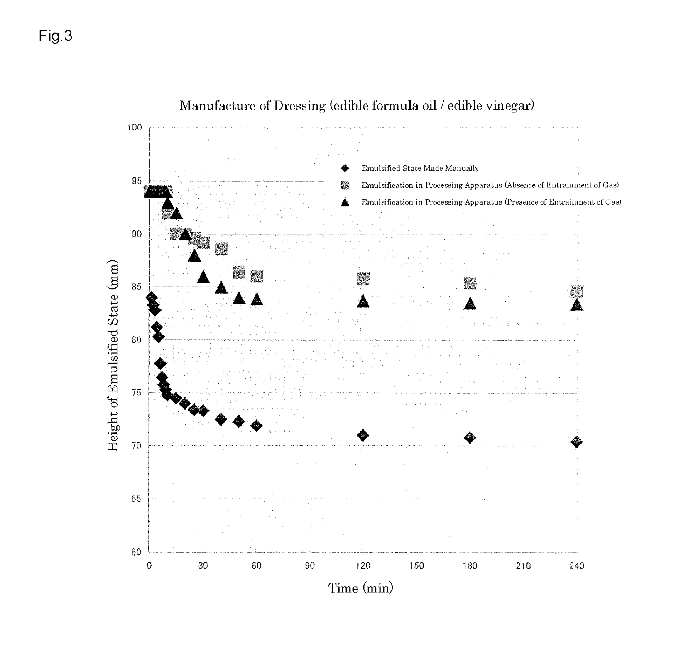

The pressurizing centrifugal pump was activated and circulated for 10 minutes without infusing air into the flow channel by an aspirator on the side of an air-inlet port of the pressurizing centrifugal pump to emulsify (emulsification of) the sample. The emulsified part is transferred from the chamber to a graduated cylinder immediately after the stop of the processing apparatus and the phase dissociated state was observed with time. The result is indicated by solid squares in Fig. 3.

Subsequently, under the same experimental conditions, the pressurizing centrifugal pump was activated and circulated for 10 minutes while infusing air into the circulating portion by an aspirator on the side of an air-inlet port of the pressurizing centrifugal pump to emulsify (emulsification of) the sample. The result is indicated by solid triangles in Fig. 3. In order to compare with the emulsification effect of the processing apparatus, the edible formula oil and the edible vinegar of an equal rate were put into a container and were shaken manually for 10 minutes hard, and the phase dissociated state of emulsified part thereafter was observed. The result is indicated by solid diamonds in Fig. 3.

The vertical axis indicates the height (mm) of the emulsified state, and the lateral axis indicates the time (minutes). Decrease in height of the emulsified state is an index which represents the degree of phase dissociation of the emulsified state (emulsion). The result of the emulsified state made manually was decreased abruptly from 95 mm to 75 mm with time and then gradually approached 70 mm. In contrast, the result of emulsification in the processing apparatus was gently decreased from 95 to 85 irrespective of the presence or absence of entrainment of gas, and then a flat state at 85 was maintained.

From the result described above, 1) the fact that the emulsified state made by the processing apparatus is hardly subject to the phase dissociation in comparison with the emulsified state made manually was suggested. 2) It became apparent that the degree of phase dissociation made by the processing apparatus was on the order of 10% after 5 to 6 hours, while the degree of phase dissociation made manually reached as much as 26%. This result indicates that the finer emulsification was achieved by the processing apparatus. 3) The changes with time were substantially the same irrespective of the presence or absence of entrainment of gas in the processing apparatus. However, the phase dissociation curve at the time when the gas is entrained appeared as a curve profile smoother than the curve in the case where the gas is not entrained. This was understood to be because that the cavitation effect worked by the entrainment of the gas to cause finer emulsification.

### <Example 6>

### Manufacture of Milk Beverage

By using the same processing apparatus as in Example 1, the chamber was filled with 12L of milk, 12L of yogurt, and 6L of grape extract, and the apparatus was activated at three-phase 200V, a frequency of 60Hz via the inverter at the number of revolutions of 4000. The circulating flow rate was set to approximately 100 L/min, the diameter of the nozzle portion of the valve was adjusted by the valve body, and the static pressure on the discharging side of the pressurizing centrifugal pump was maintained at 0.4 MPa.

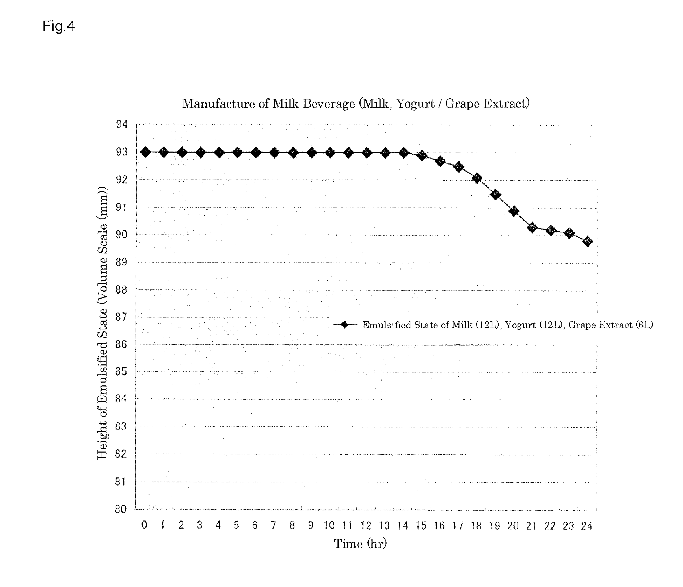

The pressurizing centrifugal pump was activated and circulated for 10 minutes while infusing air into the flow channel by an aspirator on the side of an air-inlet port of the pressurizing centrifugal pump to emulsify (emulsification of) the sample. The emulsified part is transferred to the graduated cylinder immediately after the stop of the processing apparatus and the phase dissociated state was observed with time. The result is indicated by solid diamonds in Fig. 4.

The vertical axis indicates the height (mm) of the emulsified state, and the lateral axis indicates the time (minutes). Decrease in height of the emulsified state is an index which represents the degree of phase dissociation of the emulsified state (emulsion). The height to 93 mm was maintained until 15 hours had elapsed and little phase dissociated state was recognized. However, the phase dissociation was proceeded from 93 mm to 90 mm from then on.

From the result described above, 1) the emulsified state made by the processing apparatus for the milk beverages is rarely turned into the phase dissociated state until 15 hours has elapsed. 2) It became apparent that the degree of the phase dissociation after 15 hours is as extremely low as approximately 3% of the entire amount. This result indicates that the emulsification of the milk beverage in the processing apparatus achieves sufficient refinement.

### <Example 7>

### Manufacture of Hydrogen Water and Quality Change with Time

By using the same processing apparatus as in Example 1, the chamber was filled with 30L of distilled water, and the apparatus was activated at three-phase 200V, a frequency of 60Hz via the inverter at the number of revolutions of 4000. The circulating flow rate was set to approximately 150 L/min, the diameter of the nozzle portion of the valve was adjusted by the valve body, and the static pressure on the discharging side of the pressurizing centrifugal pump was maintained at 0.5 MPa.

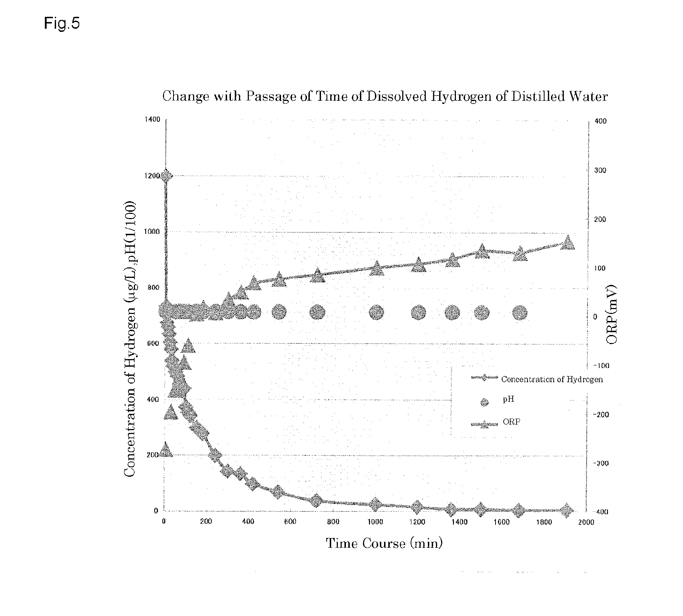

The pressurizing centrifugal pump was activated and circulated for 10 minutes while infusing hydrogen gas into the flow channel in a rate of 1 L per minute by the aspirator on the side of an air-inlet port of the pressurizing centrifugal pump to emulsify gas-liquid sample, and consequently, hydrogen water was manufactured. After the stop of the processing apparatus, changes of the amount of dissolved hydrogen, pH, and the oxidation-reduction potential (ORP) with time under the atmospheric air are monitored and the result is shown in Fig. 5. The water temperature at the time of start of the experiment was 7°C, and the water temperature at the time of end thereafter was 15°C.

Consequently, the bubble hydrogen refined by the emulsification processing apparatus (including bubble generating valve) was dissolved in water efficiently, and hydrogen water having strong reducing characters such as an amount of dissolved hydrogen of 1.2 ppm, a pH of 7.1, and an oxidation-reduction potential of approximately -500mV was obtained. The amount of dissolved hydrogen (solid diamond) was abruptly reduced in reverse proportion with time and gradually approached zero after 1500 minutes. At this time, the half-life period of the amount of dissolved hydrogen water was approximately 60 minutes. In response to the decrease in amount of dissolved hydrogen, the oxidation-reduction potential (solid triangle) was abruptly increased from the vicinity of - 500 mV until 400 minutes, and then, was increased gently, and then reached a value little smaller +200 mV after 2000 minutes. From these behaviors, it became apparent that reduction of the amount of dissolved hydrogen and increase of the oxidation-reduction potential are correlated.

In contrast, the pH value (solid circle) was kept at a constant value 7.1 even though the time had elapsed, that is, even though the dissolved hydrogen flown in all directions in the atmospheric air. The result indicates that even though the amount of dissolved hydrogen in water is reduced, the concentration of the hydrogen ion is not affected. From the result of the experiment, the reducing character of hydrogen water is estimated to be generated when the hydrogen molecule is dissolved in water by coulomb-coupling the hydrogen molecule weakly to hydron of hydron-hydril (H⁺-H⁻) and oxygen atom, thereby enhancing the effect of the hydril (-H⁻) projecting from the water molecule becomes stronger, so that the reducing character is generated.

## Claims

1. A processing apparatus for dispersion, dissolution, solubilization, or emulsification of gas/liquid or liquid/liquid configured to disperse, dissolve, solubilize, or emulsify gas into liquid or disperse, dissolve, solubilize, or emulsify liquid to liquid, comprising:
a pressurizing centrifugal pump having a pump chamber in a drum-shaped case formed with an intake port and a feed port, an impeller having a plurality of blades projecting radially from a boss portion on a side surface of a blade plate so as to have a retracted angle in the direction of rotation, a pressurizing portion having a pressurizing plane which opposes blades and forms a pressurizing chamber converging from the side of the intake port toward the side of the feed port and a pressurizing partitioning wall provided in the proximity of side surfaces of the blades and configured to prevent leakage of the fluid in an impeller chamber, the pressurizing portion opposing the impeller,
a circulating portion communicating with an intake port and a feed port of the pressurizing centrifugal pump, configuring a circulating channel together with the pressurizing centrifugal pump, and causing gas/liquid fluid or liquid/liquid fluid to be dispersed, dissolved, solubilized, or emulsified to be circulated by driving of the pressurizing centrifugal pump from the feed port to the intake port of the pressurizing centrifugal pump,
a nozzle portion provided on the downstream of the pressurizing centrifugal pump in the circulating portion and having a minute opening for allowing gas/liquid fluid or liquid/liquid fluid pressurized by the pressurizing centrifugal pump to pass therethrough, and
a chamber provided on the downstream of the nozzle portion in the circulating portion and configured to be capable of storing gas/liquid fluid or liquid/liquid fluid.

2. The processing apparatus for gas/liquid or liquid/liquid dispersion, dissolution, solubilization, or emulsification according to Claim 1, comprising a valve which can adjust the width of the minute opening of the nozzle portion by a valve body.
